# EUROPÄISCHE PATENTSCHRIFT

(11) **EP 2 164 543 B1**
(45) Veröffentlichungstag und Bekanntmachung des Hinweises auf die Patenterteilung: **17.08.2011**
(21) Anmeldenummer: 08773359.8
(22) Anmeldetag: 03.06.2008
(51) Int. Cl.: A61M 5/20

(54) **INJEKTIONSVORRICHTUNG**
INJECTION DEVICE
DISPOSITIF D'INJECTION

(30) Priorität: 08.06.2007 DE 202007008068 U; 08.09.2007 DE 202007012637 U
(43) Veröffentlichungstag der Anmeldung: 24.03.2010
(73) Patentinhaber: Bayer Schering Pharma Aktiengesellschaft, 13353 Berlin (DE)
(72) Erfinder: WEBER, Wilfried, 72296 Schopfloch (DE)
(74) Vertreter: Schön, Thilo
(86) Internationale Anmeldenummer: PCT/EP2008/004412
(87) Internationale Veröffentlichungsnummer: WO 2008/148518

(56) Entgegenhaltungen:
- WO-A-2004/054645
- WO-A-2005/011780
- WO-A-2007/033638
- US-B1- 6 270 479

## Beschreibung

### Technischer Hintergrund

Zur Behandlung einer Vielzahl von Krankheiten, die inzwischen eine große Verbreitung haben, wie z.B. der Zuckerkrankheit, muss der Patient selbständig sich die benötigte Menge eines Wirkstoffes/Arzneimittels mittels einer Spritze oder einer Karpule injizieren. Um dies sicherer und einfacher zu gestalten, sind eine Vielzahl von Injektionsvorrichtungen bekannt, die einen weitgehend automatischen Ablauf vom Einstechen der Nadel, Injizieren des Wirkstoffes und Zurückholen der Nadel beinhalten.

### Stand der Technik

Zur Verwendung von Einwegspritzen sind mehrere Vorrichtungen zur automatischen Injektion des in der Spritze enthaltenen Wirkstoffs bekannt; so zeigt die gattungsbildende WO 2007/033638 eine Injektionsvorrichtung, die bei einfacher Bedienung einen vollautomatischen Ablauf der oben beschriebenen Vorgänge bei Verwendung einer Zwei-Kammer-Ampulle ermöglicht, mit deren Hilfe eine Abfolge von Mischen, Einstechen und Injizieren ermöglicht wird.

WO 2005/011780 A2 beschreibt eine Injektionsvorrichtung mit einem Betätigungselement, mittels dem ein Injektionsvorgang einer Spritze durchgeführt werden kann. Das Betätigungselement wirkt dabei zudem mit Bauteilen zusammen, die nach Beendigung des Injektionsvorganges eine Nadel mittels eines Rückholhubes aus der Einstechstelle herausziehen. Durch den Einbau eines Dämpfungsgliedes kann dabei eine längere Injektionszeit erzielt werden. Zudem kann durch das Einsetzen eines Volumenadapters ein Umschaltpunkt zwischen einem Injektionshub und einem Leerhub verändert werden.

### Darstellung der Erfindung

Es ist Aufgabe der Erfindung, eine Injektionsvorrichtung derart weiterzubilden, dass bei einfacher mechanischer Gestaltung der Handhabungskomfort und die Sicherheit für den Patienten erhöht wird.

Die erfindungsgemäße Injektionsvorrichtung löst diese Aufgabe mit den Merkmalen des Patentanspruchs 1.

Der grundlegende Erfindungsgedanke ist darin zu sehen, dass nunmehr nicht mehr die bisher nahtlose Reihenfolge der Hübe mit fester jeweiliger Hubdauer vorgegeben ist, sondern dem Benutzer eine individuelle Gestaltung der Dauer eines Hubes (und damit der Geschwindigkeit des zugeordneten Vorgangs wie z.B. der Injektion) und auch des Übergangs einzelner Hübe (und damit insbesondere Pausen im Bewegungsablauf) ermöglicht wird.

Bevorzugte Ausgestaltung betreffen die Auslegung der Bauteileelemente zur Einstellung von Verweilzeit und Injektionsdauer.

### Kurze Beschreibung der Zeichnungen

Zwei Ausführungsbeispiele der Injektionsvorrichtung werden nun anhand von Zeichnungen näher erläutert. Es zeigen:
- Figur 1A:: Eine Seitenansicht eines ersten Ausführungsbeispiels der In- jektionsvorrichtung bei abgenommenem Gehäusedeckel,
- Figur 1 B:: einen Schnitt durch die Injektionsvorrichtung der Figur 1 in ihrer Grundstellung in ihrer Mittelebene,
- Figur 2A:: eine Seitenansicht der Injektionsvorrichtung bei der Durchfüh- rung des Mischhubes,
- Figur 2B:: eine Schnittdarstellung entsprechend Figur 2A,
- Figur 3A:: eine Seitenansicht der Injektionsvorrichtung beim Einstechhub,
- Figur 3B:: eine Schnittdarstellung gemäß Figur 3A,
- Figur 4A:: eine Seitenansicht der Injektionsvorrichtung beim Injektionshub,
- Figur 4B:: eine Schnittdarstellung gemäß Figur 4A,
- Figur 5A:: eine Seitenansicht eines ersten Ausführungsbeispiels der Injek- tionsvorrichtung beim Leerhub,
- Figur 5B:: eine Schnittdarstellung entsprechend Figur 5A,
- Figur 6A:: eine Seitenansicht eines ersten Ausführungsbeispiels der Injek- tionsvorrichtung beim Nadelrückzug,

- Figur 6B:: eine Schnittdarstellung entsprechend Figur 6A,
- Figur 7A:: eine Seitenansicht des ersten Ausführungsbeispiels der Injekti- onsvorrichtung nach Beendigung des Nadelrückzugs,
- Figur 7B:: eine Schnittdarstellung entsprechend Figur 7A,
- Figur 8A:: eine Seitenansicht eines ersten Ausführungsbeispiels der Injek- tionsvorrichtung beim Zurücksetzen der Mechanik (maximale Verweilzeit),
- Figur 8B:: eine Schnittdarstellung entsprechend Figur 8A,
- Figur 9A:: eine Seitenansicht eines ersten Ausführungsbeispiels der Injek- tionsvorrichtung beim Zurücksetzen der Mechanik (minimale Verweilzeit),
- Figur 9B:: eine Schnittdarstellung entsprechend Figur 9A,
- Figur 10A:: eine Seitenansicht eines zweiten Ausführungsbeispiels der In- jektionsvorrichtung bei abgenommenem Gehäusedeckel,
- Figur 10B:: einen Schnitt durch die Injektionsvorrichtung der Figur 1 in ihrer Grundstellung in ihrer Mittelebene 1,
- Figur 11 A:: eine Seitenansicht eines zweiten Ausführungsbeispiels der In- jektionsvorrichtung beim Einstechhub,
- Figur 11 B:: eine Schnittdarstellung entsprechend Figur 11A,
- Figur 12A:: eine Seitenansicht eines zweiten Ausführungsbeispiels der In- jektionsvorrichtung beim Injektionshub,
- Figur 12B:: eine Schnittdarstellung entsprechend Figur 12A,
- Figur 13A:: eine Seitenansicht eines zweiten Ausführungsbeispiels der In- jektionsvorrichtung beim Leerhub,
- Figur 13B:: eine Schnittdarstellung entsprechend Figur 13A,
- Figur 14A:: eine Seitenansicht des zweiten Ausführungsbeispiels der Injek- tionsvorrichtung beim Nadelrückzug,
- Figur 14B:: eine Schnittdarstellung entsprechend Figur 14A,
- Figur 15A:: eine Seitenansicht des zweiten Ausführungsbeispiels der Injek- tionsvorrichtung nach Beendigung des Nadelrückzugs,
- Figur 15B:: eine Schnittdarstellung entsprechend Figur 15A,

- Figur 16A:: eine Seitenansicht des zweiten Ausführungsbeispiels der Injek- tionsvorrichtung beim Zurücksetzen der Mechanik maximaler Verweilzeit,
- Figur 16B:: eine Schnittdarstellung entsprechend Figur 16A,
- Figur 17A:: eine Seitenansicht des zweiten Ausführungsbeispiels der Injek- tionsvorrichtung beim Zurücksetzen der Mechanik minimaler Verweilzeit,
- Figur 17B:: eine Schnittdarstellung entsprechend Figur 17A,
- Figur 18A:: eine Seitenansicht eines ersten Ausführungsbeispiels eines Planetengetriebes mit einem Rotationsdämpfungsglied,
- Figur 18B:: eine Schnittdarstellung entsprechend Figur 18A,
- Figur 18C:: eine Ansicht des Planetengetriebes ohne Planetenradträger,
- Figur 18D:: eine perspektivische Darstellung entsprechend Figur 18A mit gesperrtem Planetenradträger,
- Figur 18E:: eine perspektivische Darstellung entsprechend Figur 18C ohne Planetenradträger,
- Figur 18F:: eine Ansicht entsprechend Figur 18D mit gesperrtem Planeten- radträger,
- Figur 19A:: eine Seitenansicht eines zweiten Ausführungsbeispiels eines Planetengetriebes mit zwei Rotationsdämpfungsgliedern,
- Figur 19B:: eine Seitenansicht und eine perspektivische Darstellung nach Figur 19A mit gesperrter erster Sperrscheibe,
- Figur 19C:: eine Seitenansicht und eine perspektivische Darstellung ent- sprechend Figur 19B mit gesperrter zweiter Sperrscheibe,
- Figur 19D:: eine perspektivische Darstellung nach Figur 19B mit gesperrter erster Sperrscheibe,
- Figur 19E:: eine perspektivische Darstellung nach Figur 19D ohne Plane- tenradträger/zweiter Sperrscheibe,
- Figur 19F:: einen Schnitt in der Ebene B-B der Figur 19A,
- Figur 19G:: einen ersten Schnitt in der Ebene A-A der Figur 19A, und
- Figur 19H:: einen zweiten Schnitt in der Ebene A-A der Figur 19A.

### Beschreibung des ersten Ausführungsbeispiels

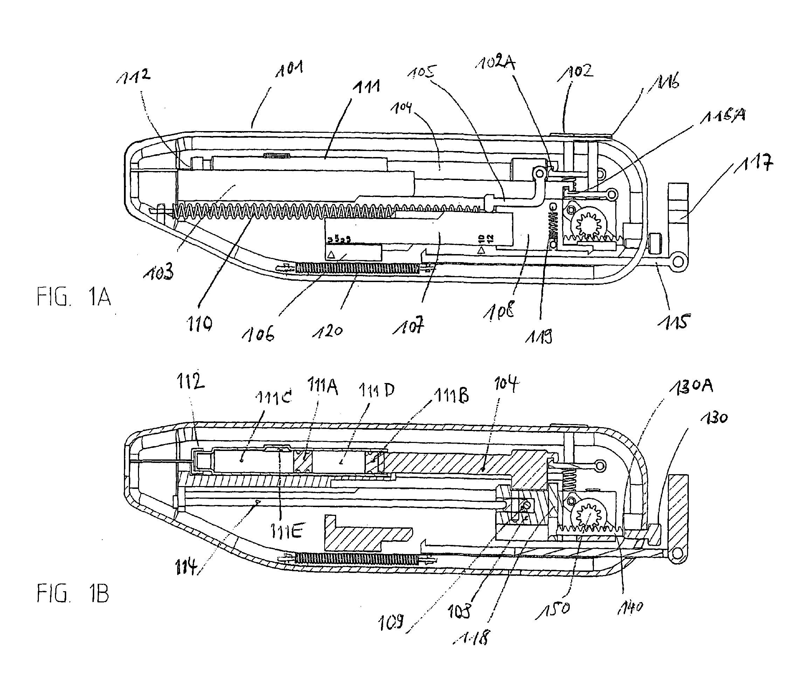

Zur Injektion des Wirkstoffes wird eine Zweikammer-Ampulle 111 verwendet. Eine solche Ampulle (Figur 1 B) hat zwei Kolben 111 A, 111 B, dadurch ergeben sich zwei zunächst voneinander unabhängige Kammern 111C, 111 D. In die erste, innere Kammer 111C, die der Kanüle 112 zugewandt ist, wird zum Beispiel Betaferon in Pulverform, in die zweite, äussere Kammer 111 D eine NaCl - Lösung eingefüllt.

Wird nun ein Stössel 104 gegen den äußeren Kolben 111 B gedrückt, verschiebt sich zunächst auch der innere Kolben 111 A, da die NaCl - Lösung die Kraft des Stössels hydraulisch auf den inneren Kolben 111 A überträgt. Sobald der innere Kolben 111 A einen Überströmkanal 111E in Form einer nutförmigen Ausbuchtung im Mantel der Ampulle 111 überlaufen hat, bleibt dieser stehen und die NaCl- Lösung strömt über diesen Überströmkanal 111 E in die innere Kammer 111C und mischt sich mit dem Betaferon. Nach dem Mischen erfolgt dann (nach dem Einstechhub) die Injektion durch das Weiterbewegen des Stössels 104.

Figur 1A zeigt eine Aufsicht, Figur 1B einen Schnitt in der Ausgangsstellung der Injektionsvorrichtung.

Sämtliche Bauteile befinden sich in einem Gehäuse 101, das aus zwei wannenförmigen Halbschalen besteht. Die Bewegungsbauteile sind hierbei in der Injektionsvorrichtung parallel zur Längsachse der Nadel verschiebbar gehalten. Die Bauteile sind wie folgt einander zugeordnet:
Die Zweikammer-Ampulle 111 ist in einer Aufnahme 103 gehalten. Ein Stössel 104, an dessen hinterem Ende ein Steuerhebel 105 angelenkt ist, ist an einem Rasthaken 102A einer federbeaufschlagten ersten Taste 102 gehalten. Die Aufnahme 103 ist an einem Rasthaken 116A einer federbeaufschlagten zweiten Taste 116 gehalten.
Auf die Aufnahme 103 wirkt das Ende eines Zugseils 114, welches über eine in einem Schlitten 108 gelagerte Rolle 109 umgelenkt wird, und mit einer Zugfeder 110 verbunden ist, die am Gehäuse 101 befestigt ist. Die Zugfeder 110 übt somit einen Zug auf die Aufnahme 103 in entgegengesetzter Richtung zur Einstichstelle aus. Die Aufnahme 103 kann sich jedoch nicht axial verschieben, da sie vom Rasthaken 116A an der zweiten Taste 116 gehalten wird.

Durch die Umlenkung des Zugseiles 114 über die Rolle 109 entsteht eine Kraft auf den Schlitten 108 in Richtung der Einstichstelle. Der Schlitten 108 bleibt jedoch in seiner Position, da er über einen im Schlitten 108 senkrecht zur Injektionsrichtung verschiebbar gelagerten von einer Mitnehmerfeder 119 beaufschlagten Mitnehmer 118 am Stössel 104 anliegt und der Stössel 104 vom Rasthaken 102A an der ersten Taste 102 gehalten wird.

Dem Steuerhebel 105 ist ein erster Einstellschieber 107 zugeordnet, in dem ein zweiter Einstellschieber 106 verschiebbar gelagert ist. Der Einstellschieber 106 dient zur Entkoppelung des Schlittens 108 vom Stössel 104. Die Einstellschieber 106,107 sind als verschiebbar gelagerte Anschlagelemente zur Einstellung von Einstechtiefe und Injektionsvolumen ausgebildet, wie dies weiter unten noch erläutert wird.

Ein Rückzuggriff 117, der mit einer Zugstange 115 verbunden ist, dient zur Herstellung dieser Ausgangslage. Die Zugstange 115 wird durch eine Rückzugfeder 120 beaufschlagt.

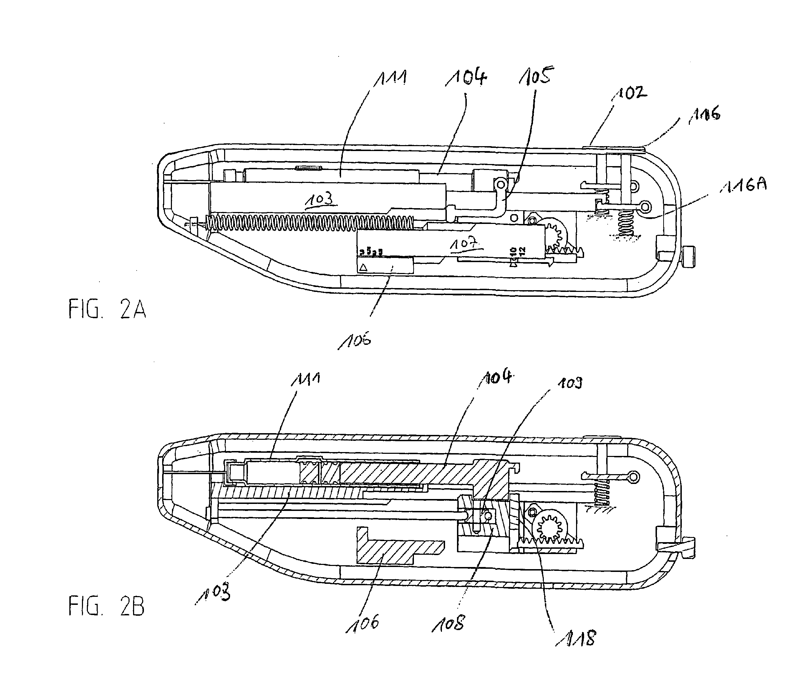

Wird die erste Taste 102 betätigt, kommt der Rasthaken 102A außer Eingriff, der Stössel 104 wird freigegeben und bewegt sich in Richtung Einstichstelle, bis die Vorderkante des Steuerhebels 105 an der Aufnahme 103 anliegt. Auf diese Weise wird der äußere Kolben 111 B der Ampulle 111 beaufschlagt, bewegt sich nach vorne und führt einen Mischhub H0 durch. Dieser Mischhub dient dem Mischen der NaCl-Lösung mit dem Betaferon wie oben beschrieben (Figur 2A, Figur 2B). Durch ein Sichtfenster im Gehäuse 101 kann die Vermischung des Betaferons mit der NaCl-Lösung kontrolliert werden.

Da das freie Ende des Steuerhebels 105 andererseits auf dem zweiten Einstellschieber 107 gleitet und dort aufliegt, kann er an dieser Stelle nicht durch Verschwenken nach unten ausweichen; somit wird die Zugkraft der Zugfeder 110 in Richtung Einstichstelle vom Schlitten 108 über den Stössel 104 auf die Aufnahme 103 übertragen. Die Aufnahme 103 bleibt jedoch in ihrer Lage, da sie vom Rasthaken 116A der Taste 116 arretiert ist.

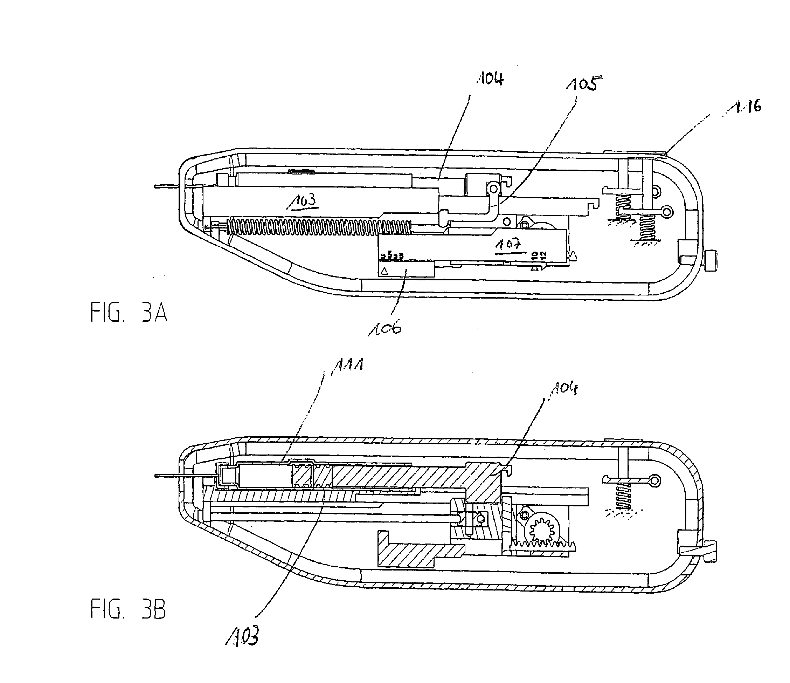

Wird nun die zweite Taste 116 betätigt, kommt der Rasthaken 116A außer Eingriff und die Aufnahme 103 wird freigegeben; dadurch bewegen sich der Stössel 104 und die Aufnahme 103 unter der Wirkung der Zugfeder 110 gemeinsam in Richtung Einstichstelle. Die Nadel wird eingestochen (Figur 3A,3B), der Einstechhub H1 wird durchgeführt.

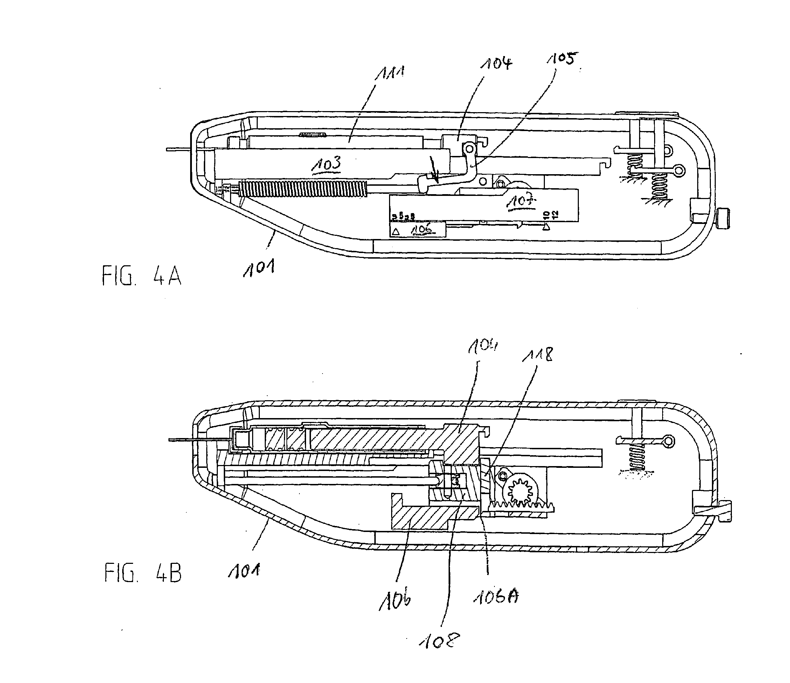

Ist die gewünschte Einstechtiefe erreicht, kann der Steuerhebel 105 nach unten schwenken (Pfeil in Figur 4A), da er nicht mehr durch den ersten Einstellschieber 107 wegen dessen zurückspringender Oberfläche daran gehindert wird. Es erfolgt somit keine Kraftübertragung mehr vom Stössel 104 auf die Aufnahme 103, die Aufnahme 103 verbleibt in ihrer Position, nur der Stössel 104 bewegt sich weiter zur Einstichstelle hin, d. h. die Injektion des Arzneimittels erfolgt, der Injektionshub H2 wird durchgeführt.

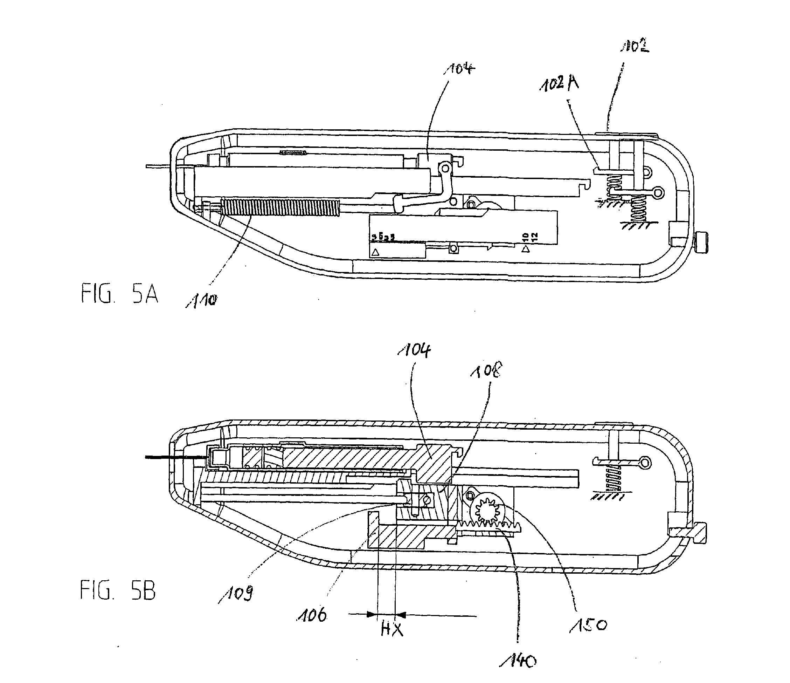

Erreicht der im Schlitten 108 verschiebbar gelagerte Mitnehmer 118 die Rampe 106A des zweiten Einstellschiebers 106 (Figur 4B), wird der Mitnehmer 118 nach unten gezogen und somit der Schlitten 108 vom Stössel 104 entkoppelt, d. h. zu diesem Zeitpunkt wird die Injektion beendet (Figur 5B).

In der Figur 5 ist eine Zahnstange 140, die mittels einer ersten Einstellschraube 130 im Gehäuse zwischen einer vorderen und einer hinteren Endposition vom Benutzer frei verschiebbar gelagert ist, in ihrer vorderen Endposition dargestellt, in der die maximale Verweilzeit (maximale Dauer des Leerhubs HX) eingestellt ist.

Ist die Injektion beendet, trifft die Zahnstange 140 auf den zweiten Einstellschieber 106, der Schlitten 108 bewegt sich gemeinsam mit einem mit dem Schlitten 108 verbundenen Dämpfungsglied 150 weiter in Richtung der Einstechstelle relativ zur Zahnstange 140, wodurch der Leerhub HX entsteht, während dem die Nadel in der Einstichstelle verbleibt. Durch die Relativbewegung zwischen Zahnstange 140 und Dämpfungsglied 150 während des Leerhubes HX ist das Dämpfungsglied 150 wirksam. Trifft der Schlitten 108 auf den zweiten Einstellschieber 106, ist der Leerhub HX beendet.

Ist die Zahnstange 140 nicht in ihrer vorderen Endosition, trifft sie zu einem späteren Zeitpunkt auf den zweiten Einstellschieber 106, und somit ist die Zeit, in der das Dämpfungsglied 150 wirksam ist, kleiner und damit auch die Verweilzeit (Dauer des Leerhubs HX).

Ist die Verweilzeit auf den minimalen Wert eingestellt, trifft die Zahnstange 140 erst nach Beendigung des Leerhubes HX auf den Schlitten 108 und wird gegenüber dem Schlitten 108 bzw. dem Dämpfungsglied 150 nicht verschoben, das Dämpfungsglied 150 ist nicht wirksam und beeinflusst dadurch die Verweilzeit somit nicht.

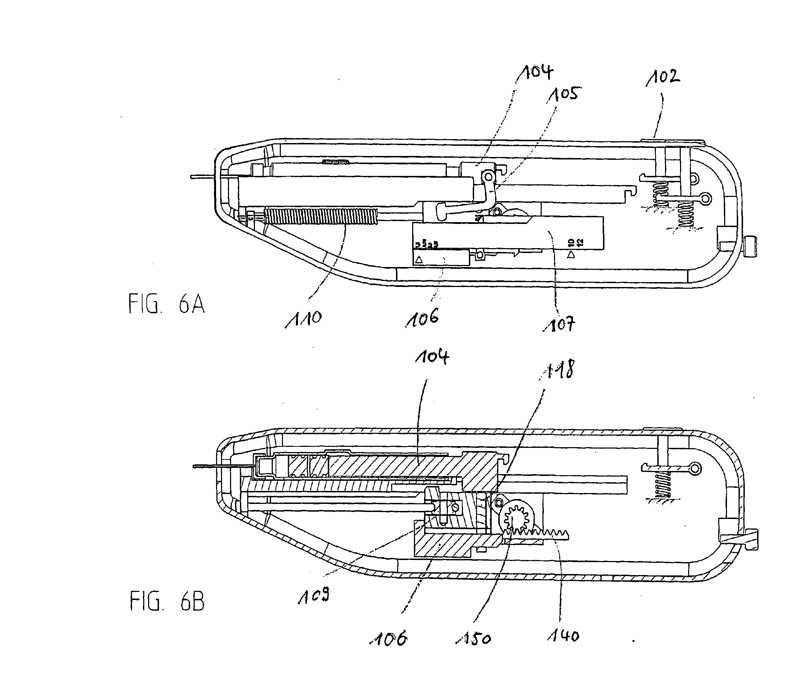

Der Schlitten 108 steht nun am zweiten Einstellschieber 106 an. Da der zweite Einstellschieber 106 über den ersten Einstellschieber 107 formschlüssig am Gehäuse 101 gehalten wird, wirkt nun die Zugkraft der Zugfeder 110 (die am Gehäuse 101 befestigt ist) über die Rolle 109 auf die Aufnahme 103, die dadurch zurückgezogen wird und damit die Nadel aus der Einstichstelle zieht (Figur 6A, 6B), der Rückholhub H3 wird durchgeführt.

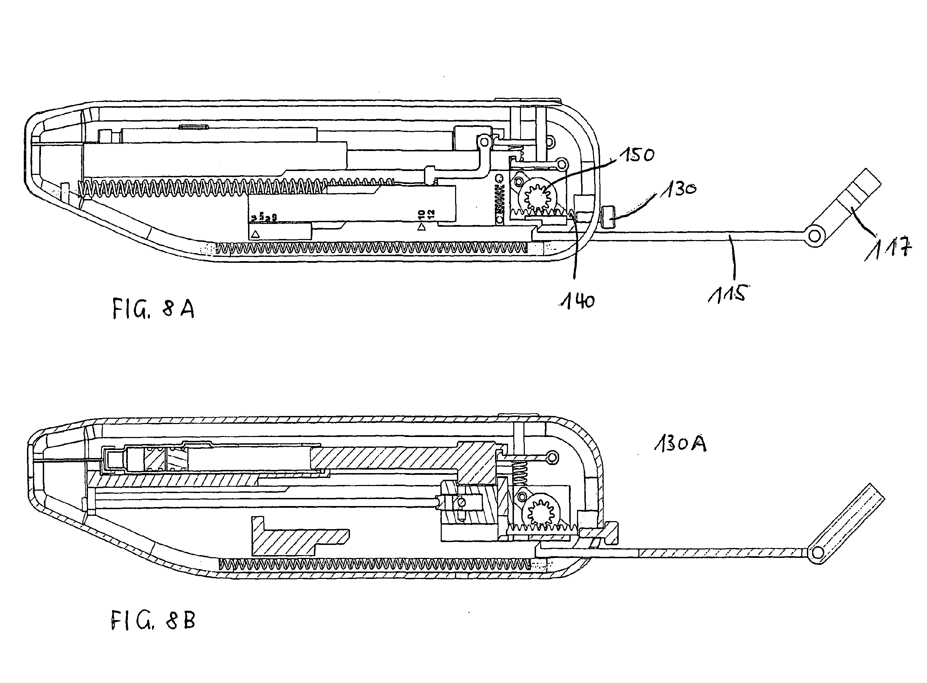

Durch Herunterklappen des Rückzuggriffes 117, der mit der Zugstange 115 verbunden ist, und Herausziehen der Zugstange 115 werden Schlitten 108 und alle anderen Elemente wieder in die Ausgangslage (Figur 8A,8B) zurückgezogen.

Beim Zurückziehen des Schlittens/Dämpfers in die Ausgangslage trifft die Zahnstange 140 auf die stirnseitige erste Anschlagfläche 130A der Einstellschraube 130. Während sich der Schlitten 108 mit dem Dämpfungsglied 150 weiter von der Einstechstelle weg bewegen, wird die Zahnstange 140 durch die Einstellschraube 130 in der Position gehalten, d.h. die Zahnstange 140 bewegt sich relativ zum Schlitten 108 / Dämpfungsglied 150 in die eingestellte Ausgangslage.

Durch die Einstellschraube 130 kann die Lage der Fläche 130A verändert werden und damit die Strecke, die sich die Zahnstange 140 relativ zum Schlitten 108/Dämpfungsglied 150 verschiebt, eingestellt werden.

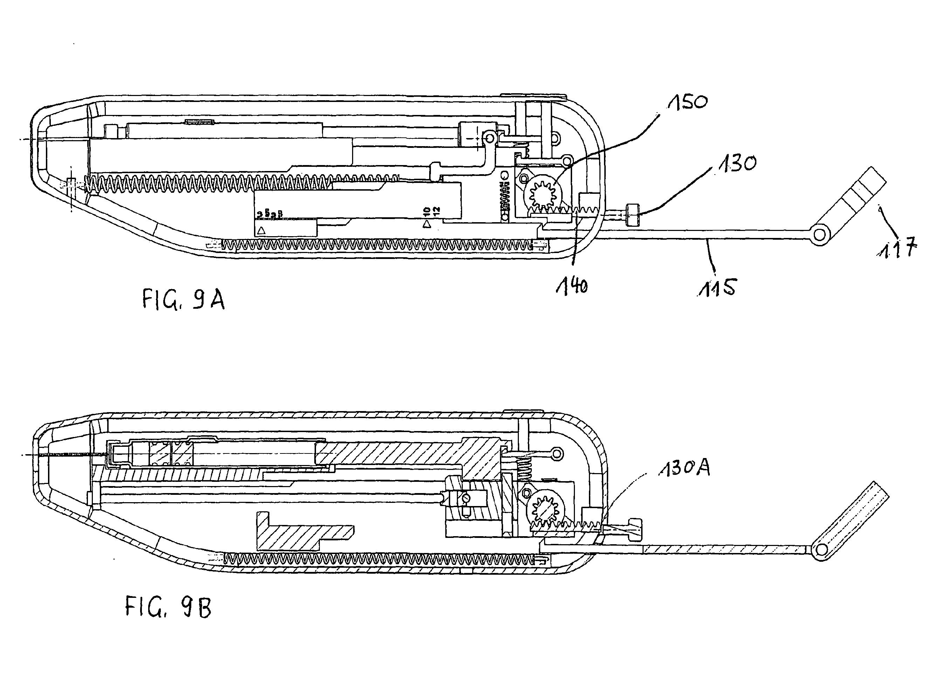

In Figur 8A/8B ist die Einstellschraube 130 in der Position dargestellt, die die maximale Verweilzeit ergibt. In Figur 9A/9B ist die Einstellschraube 130 in der Position dargestellt, die die minimale Verweilzeit ergibt. Zwischen diesen beiden Positionen lässt sich die Verweilzeit stufenlos einstellen.

Nachdem die Mechanik wieder in die Ausgangsstellung gebracht ist, kann die Karpule nun entnommen werden.

Das Injektionsvolumen und die Einstechtiefe lassen sich wie folgt einstellen:
Der erste Einstellschieber 107 ist im Gehäuse 101 axial verschiebbar gelagert, er hat in diesem Beispiel 2 Rastpositionen (10 und 12 mm, im Beispiel auf 10 mm eingestellt). Diese Rastpositionen sind dem Einstechhub H1 zugeordnet, da die axiale Lage des Einstellschiebers 107 den Weg bestimmt, bis der Steuerhebel 105 den Stössel 104 von der Aufnahme 103 entkoppelt (Figur 2A).
Im ersten Einstellschieber 107 ist der zweite Einstellschieber 106 ebenfalls axial verschiebbar mit im Beispiel 4 Rastpositionen gelagert (1.0; 0,75; 0,5; 0,25, im Beispiel auf 1,0 eingestellt). Diese Rastpositionen sind dem Injektionshub H2 zugeordnet, da die axiale Lage des Einstellschiebers 106 den Weg bestimmt, bis der Stössel 104 vom Schlitten 108 entkoppelt wird (Figur 5A,5B) und der Nadelrückzug erfolgt.

Soll nun z. B. eine Einstechtiefe von 12 mm eingestellt werden, muss gegenüber dem dargestellten Zustand der erste Einstellschieber 107 um 2 mm in Richtung der Einstechstelle in die neue Rastposition am Gehäuse 101 verschoben werden. Da der zweite Einstellschieber 106 mit dem ersten Einstellschieber 107 in der Position 1,0 verrastet ist, verschiebt sich auch dieser um 2 mm zur Einstechstelle hin, d. h., die Einstellung einer anderen Einstechtiefe wirkt sich nicht auf die Einstellung des Injektionsvolumens aus. Ebenso wirkt sich die Einstellung des Injektionsvolumens nicht auf Einstechtiefe aus; die Einstellungen von Einstechhub H1 und Injektionshub H2 sind unabhängig voneinander.

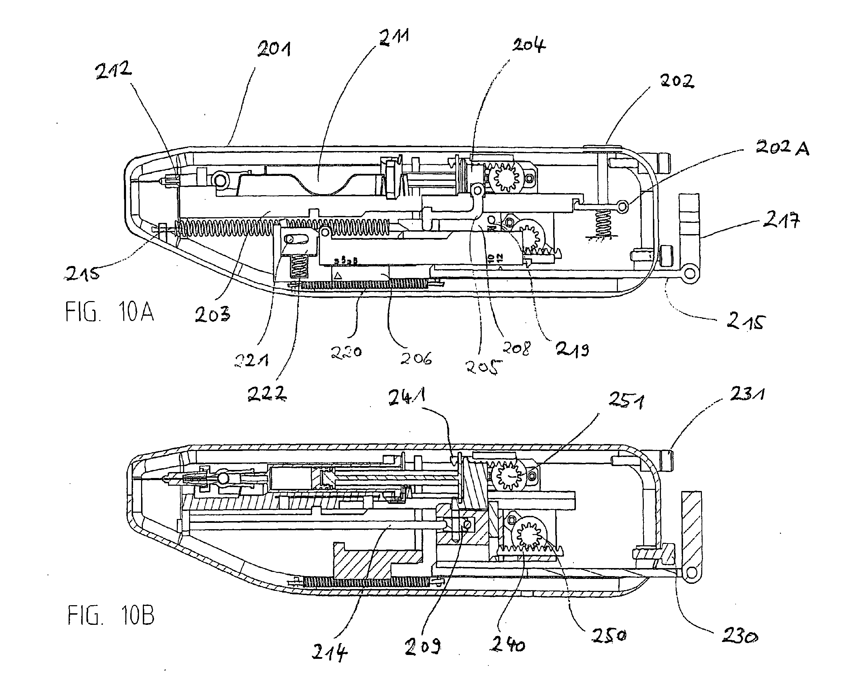

Das in Figur 10-17 dargestellte zweite Ausführungsbeispiel geht von einer Injektionsvorrichtung aus, wie im ersten Ausführungsbeispiel beschrieben, jedoch nicht für die Verwendung einer Karpule, sondern einer Spritze, d.h. der Mischhub entfällt. Der Grundgedanke dieses Ausführungsbeispiels ist, dass die Injektionszeit und/oder die Verweilzeit vom Patienten eingestellt werden kann.

Aufbau und Zusammenwirken der beim ersten Ausführungsbeispiel erläuterten Komponenten sind funktionsgleich, so dass im folgenden nur die zusätzlichen Bauteile anhand ihrer Funktionen beschrieben werden:

### Einstellung der Dauer des Injektionshubs:

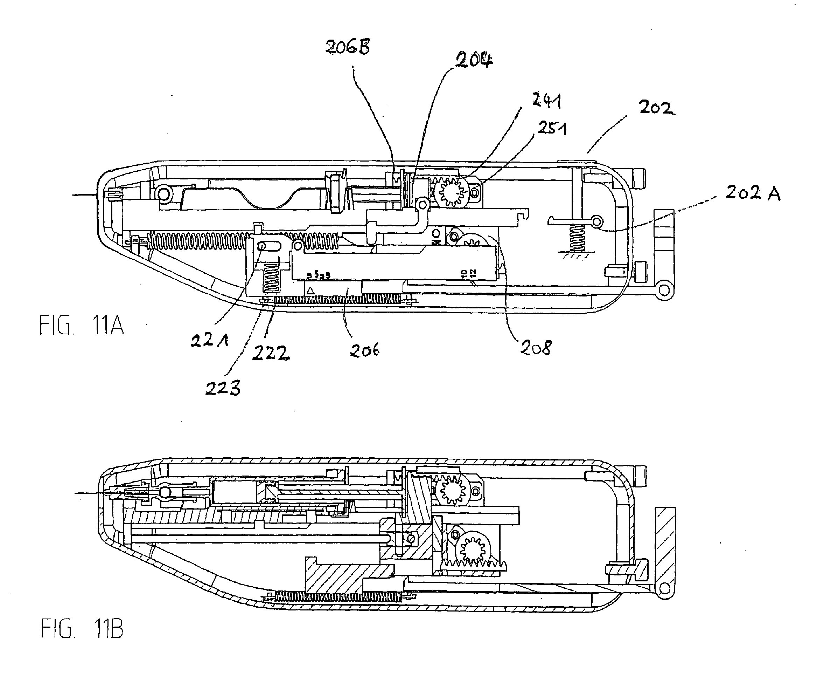

Wird die erste Taste 202 betätigt, kommt der Rasthaken 202 A außer Eingriff und die Aufnahme 203 wird freigegeben; dadurch bewegen sich der Stössel 204 und die Aufnahme 203 unter Wirkung der Zugfeder 210 gemeinsam in Richtung der Einstechstelle. Die Nadel wird eingestochen (Figur 11A und 11B), der Einstechhub wird ausgeführt.

Mit Beendigung des Einstechhubes kann eine Arretierung 221, welche über ein Langloch in einer Führung 222 eine Kraft in Richtung der Aufnahme (ausgelöst durch eine Feder 223) erfährt, in die Aussparung der Aufnahme 203 schwenken. Gelagert ist die Arretierung im ersten Einstellschieber 207 und ist somit unabhängig von der eingestellten Einstechtiefe. Die Aufnahme 203 ist über die Arretierung 221 in der eingestochenen Position fixiert.

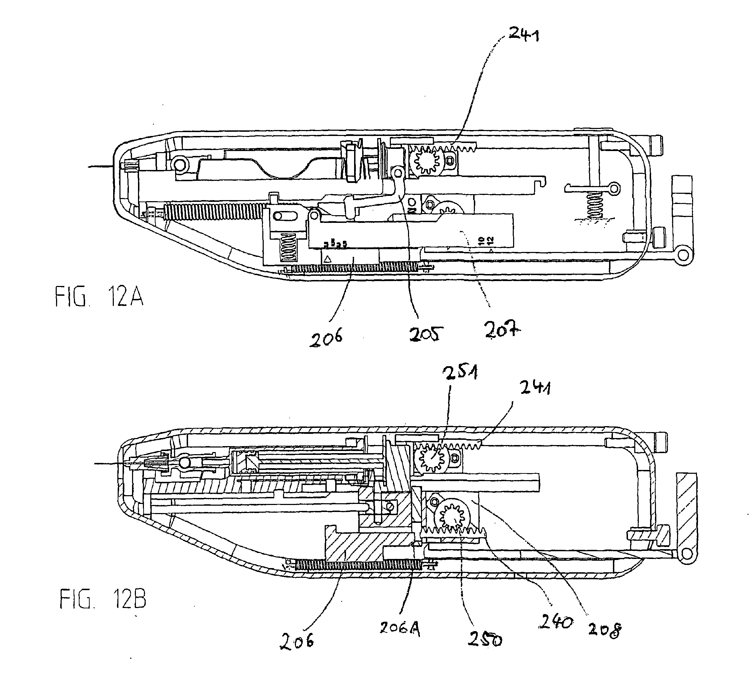

Ist die gewünschte Einstechtiefe erreicht, kann der Steuerhebel 205 nach unten schwenken (Pfeil in Figur 12A), da er nicht mehr durch den ersten Einstellschieber 207 wegen dessen zurückspringener Oberfläche daran gehindert wird.

Es erfolgt somit keine Kraftübertragung mehr auf den Stössel 204.

In Figur 11A ist der Zustand der Injektionsvorrichtung bei maximaler Injektionszeit dargestellt, d.h. eine Zahnstange 241 trifft gleichzeitig mit Ende des Einstechhubs auf einen Anschlag 206B des zweiten Einstellschiebers 206.

Ist die Injektionszeit auf einen kleineren Wert eingestellt, trifft die Zahnstange 241 zu einem späteren Zeitpunkt auf den Anschlag 206A.

Nachdem der Stössel 204 von der Aufnahme 203 entkoppelt ist, verbleibt die Aufnahme 203 in ihrer Position und der Stössel 204 bewegt sich weiter Richtung Einstechstelle. Ab dem Zeitpunkt, in dem die Zahnstange 241 auf den Anschlag 206B des zweiten Einstellschiebers 206 trifft, bleibt diese relativ zum Stössel 204 bzw. zu einem Dämpfungsglied 251 stehen. Es entsteht eine Relativbewegung zwischen Zahnstange 241 und Dämpfungsglied 251, und somit wird der Injektionshub durch das Dämpfungsglied 251 gebremst. Die Injektionszeit ist somit abhängig von der Strecke, über die das Dämpfungsglied 251 wirksam ist, und diese Strecke lässt sich über eine Einstellschraube 231 vom Patienten einstellen. Die Injektion des Arzneimittels erfolgt, der Injektionshub wird ausgeführt.

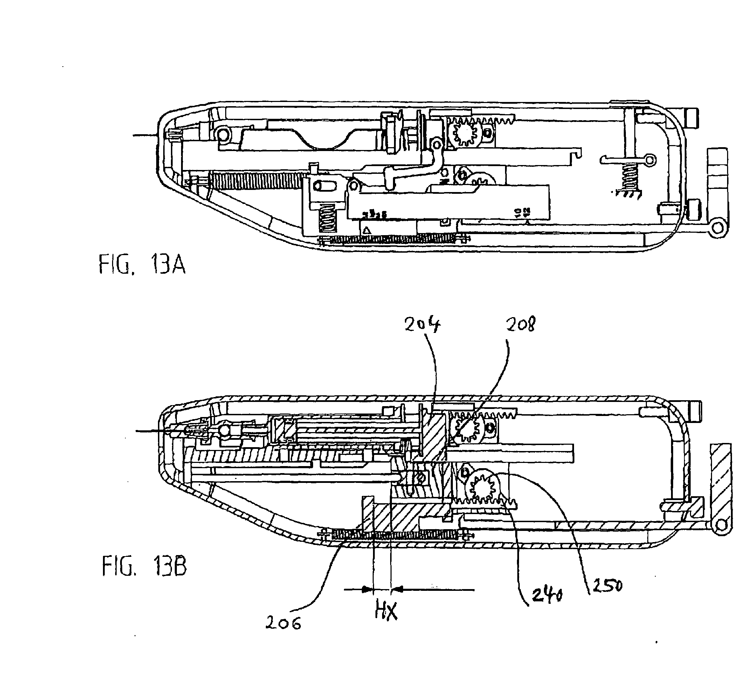

Erreicht der im Schlitten 208 verschiebbar gelagerte Mitnehmer 218 die Rampe 206A des Einstellschiebers 206 (Figur 12b), wird der Mitnehmer 218 nach unten gezogen und somit der Schlitten 208 vom Stössel 204 entkoppelt, d.h. zu diesem Zeitpunkt ist die Injektion beendet. (Figur 13b).

### Einstellung der Verweilzeit:

In der Figur 13B ist die Zahnstange 240 in ihrer vordersten Position dargestellt, d.h. es ist die maximale Verweilzeit eingestellt. Ist die Injektion beendet, trifft die Zahnstange 240 auf den zweiten Einstellschieber 206, der Schlitten 208 bewegt sich gemeinsam mit dem Dämpfungsglied 250 weiter in Richtung der Einstechstelle relativ zur Zahnstange 240. Durch die Relativbewegung zwischen Zahnstange 240 und Dämpfungsglied 250 während des Leerhubes HX ist das Dämpfungsglied 250 wirksam. Trifft der Schlitten 208 auf den zweiten Einstellschieber 206, ist der Leerhub HX beendet.

Ist die Zahnstange 240 nicht in der vordersten Position (kleinere Verweilzeit), trifft sie zu einem späteren Zeitpunkt auf den zweiten Einstellschieber 206 und somit ist die Strecke, in der das Dämpfungsglied 250 wirksam ist, kleiner und damit auch die Verweilzeit.

Ist die Verweilzeit auf den minimalen Wert eingestellt, trifft die Zahnstange 240 erst nach Beendigung des Leerhubes HX auf den Schlitten 208 und wird gegenüber dem Schlitten 208 bzw. dem Dämpfungsglied 250 nicht verschoben, das Dämpfungsglied 250 ist nicht wirksam und beeinflusst die Verweilzeit nicht.

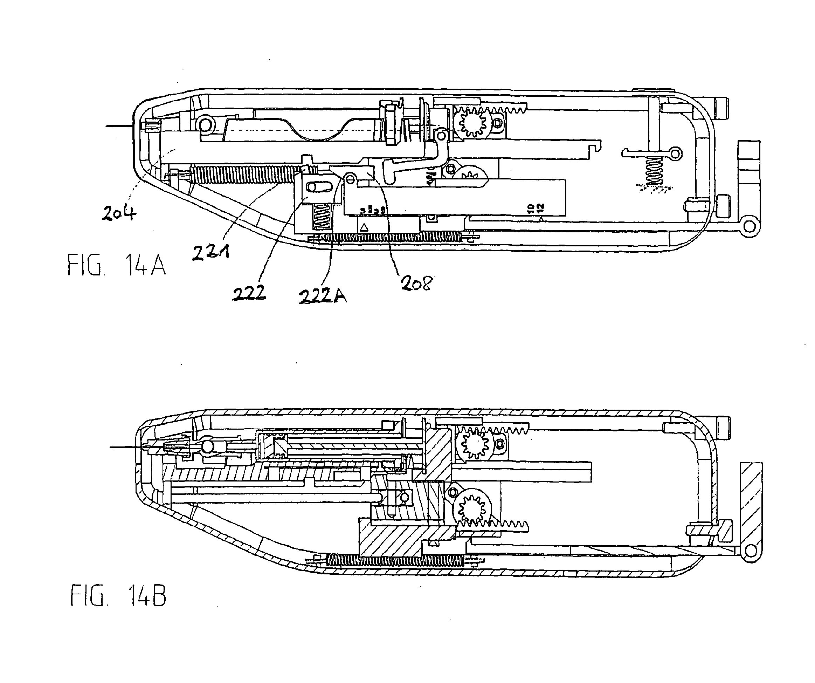

Am Ende des Leerhubes trifft die Schräge des Schlittens 208 auf eine Rampe 222A einer Führung 222 und löst die Arretierung zwischen Aufnahme 203 und Einstellschieber 206 (Figur 14A).

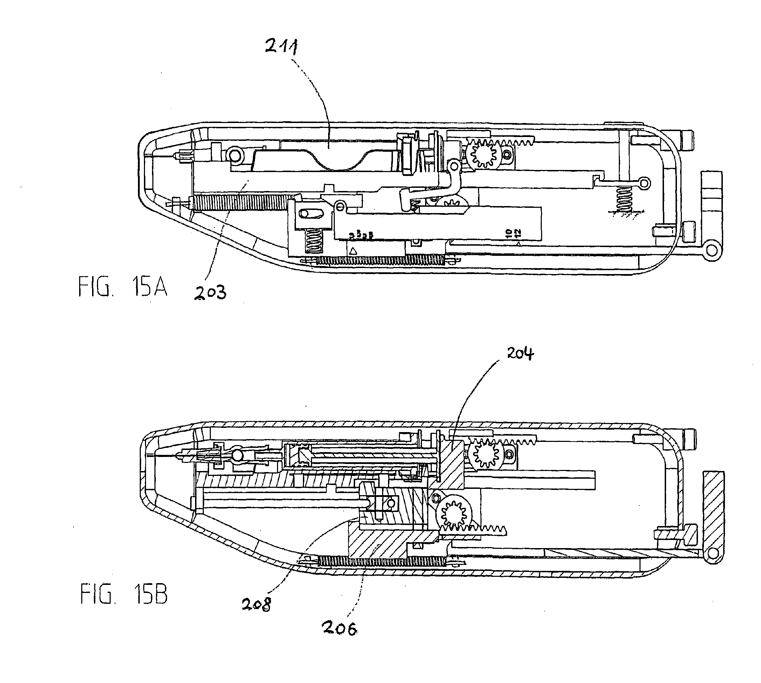

Der Schlitten 208 steht nun am zweiten Einstellschieber 206 an. Da der zweite Einstellschieber 206 über den ersten Einstellschieber 207 formschlüssig am Gehäuse 201 gehalten wird, wirkt nun die Zugfeder 210 (die am Gehäuse 201 befestigt ist) über die Rolle 209 auf die Aufnahme 203, die dadurch zurückgezogen wird und damit die Nadel aus der Einstichstelle zieht (Figur 15A,15B), der Rückhub H3 wird durchgeführt.

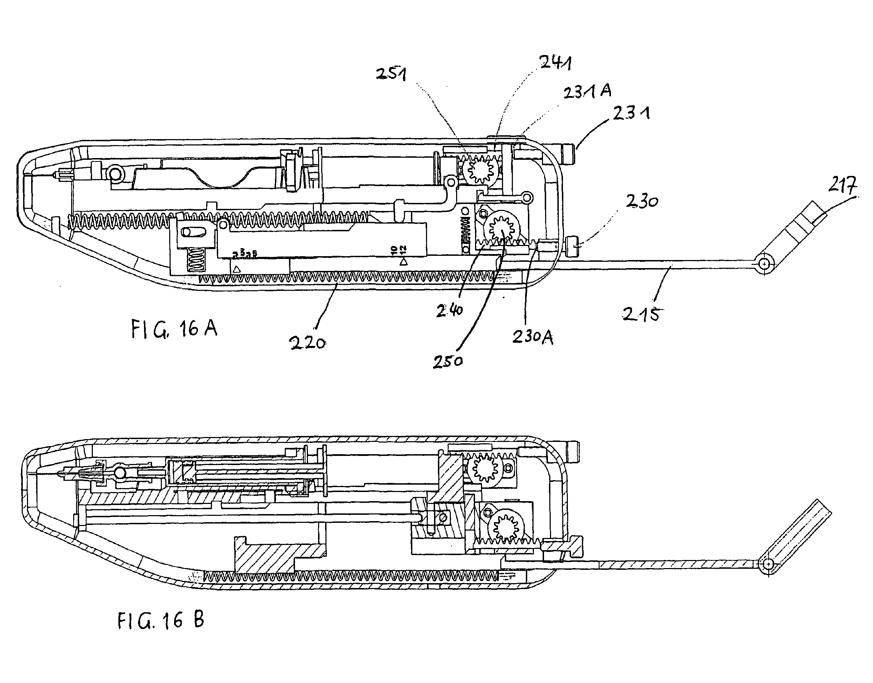

Durch Herunterklappen des Rückzugriffes 217, der mit der Zugstange 215 verbunden ist, und Herausziehen der Zugstange 215, werden der Schlitten 208 und alle anderen Elemente wieder in die Ausgangsstellung zurückgezogen (Figur 16A,16B).

Beim Zurückziehen des Schlittens 208/Dämpfungsgliedes 250 bzw. Stössel 204/Dämpfungsglieds 251 in die Ausgangslage, trifft die Zahnstange 240 auf die Fläche 230A der Einstellschraube 230 bzw. die Zahnstange 251 auf die Fläche 231A der Einstellschraube 231. Während sich Schlitten 208/Dämpfungsglied 250 bzw. Stössel 204/Dämpfungsglied 251 weiter von der Einstechstelle weg bewegen, wird die Zahnstange 240 durch die Einstellschraube 230 bzw. die Zahnstange 241 durch die Einstellschraube 231 in der Position gehalten, d.h. die Zahnstangen 240/241 bewegen sich relativ zu den Dämpfungsgliedern 250/251 in die eingestellte Ausgangslage.

Durch die Einstellschrauben 230/231 kann die Lage der Anschlagflächen 230A und 231A verändert werden. Die Strecken, die sich die Zahnstangen 240/241 relativ zu den Dämpfungsgliedern 250/251 verschieben, kann vom Patienten eingestellt werden.

In Figur 16A/16B sind die Einstellschrauben 230 und 231 in der Position dargestellt, die zur maximalen Verweilzeit/Injektionszeit führt.

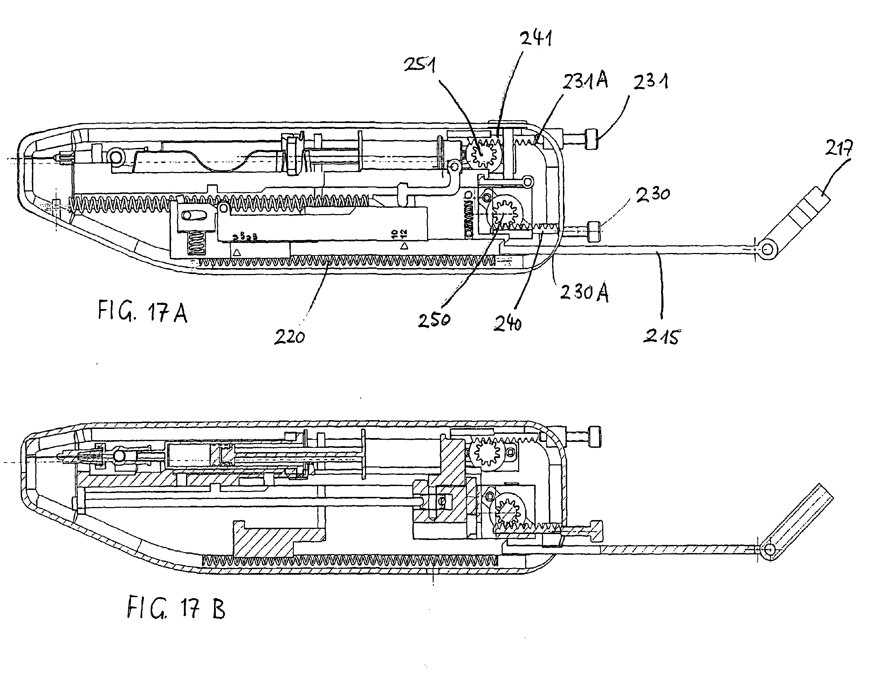

In Figur 17A/17B sind die Einstellschrauben 230 und 231 in der Position dargestellt, die die minimale Verweilzeit/Injektionszeit ergeben. Zwischen diesen beiden Positionen lassen sich Verweilzeit (Dauer des Leerhubs HX) und Injektionszeit (Dauer des Injektionshubs H2) voneinander unabhängig stufenlos einstellen.

Nachdem die Mechanik wieder in die Ausgangsstellung gebracht ist, kann die Spritze 211 nun entnommen werden.

Bei den beschriebenen Ausführungsbeispielen ist es zur Einstellung der Dauer des Injektionshubs durch entsprechende Auslegung des Dämpfungsglieds somit dem Benutzer möglich, mittels Verschiebung der zugeordneten Zahnstange die Wirkungsdauer des Dämpfungsgliedes innerhalb eines Hubs einzustellen, so dass ein Teil des Hubs ungedämpft, der verbleibende Teil gedämpft abläuft.

Alternativ kann auch ein Dämfungsglied Verwendung finden, dessen Dämpfungscharakteristik vom Benutzer einstellbar ist, um die Hubdauer zu variieren.

Auch Kombinationen solcher Maßnahmen zur Erzeugung einer gewünschten Ablaufcharakteristik (Geschwindigkeitsprofil) eines Hubes sind möglich.

Zur Realisierung dieser Alternativen zur benutzerdefinierten Einstellung von Geschwindigkeit/Dauer eines Hubs, beispielsweise des Injektionshubes, kann in den beschriebenen Ausführungsbeispielen ein Rotationsdämpfungsglied handelsüblicher Bauart verwendet werden.

Vorteilhafterweise kann ein Rotationsdämpfungsglied eingesetzt werden, wie es in der DE 20 2006 017 578.3 U1 beschrieben ist. Mit letzterem ist es möglich, eine Grunddämpfung einzustellen, was eine noch flexiblere Anpassung des Ablaufprofils eines Hubes an die individuellen Bedürfnisse des Benutzers ermöglicht.

Wenn bereits durch den Einstellbereich dieser Grunddämpfung eines Rotationsdämpfungsglieds nach der DE 20 2006 017 578.3 U1 die gewünschte Variation der Dauer des betreffenden Hubes erreichbar ist, kann ggf. auf die Einstellung der zugehörigen Zahnstange mittels ihrer zugeordneten Einstellschraube verzichtet werden und diese in der Position verbleiben, in der sie die maximale Hubdauer ermöglicht.

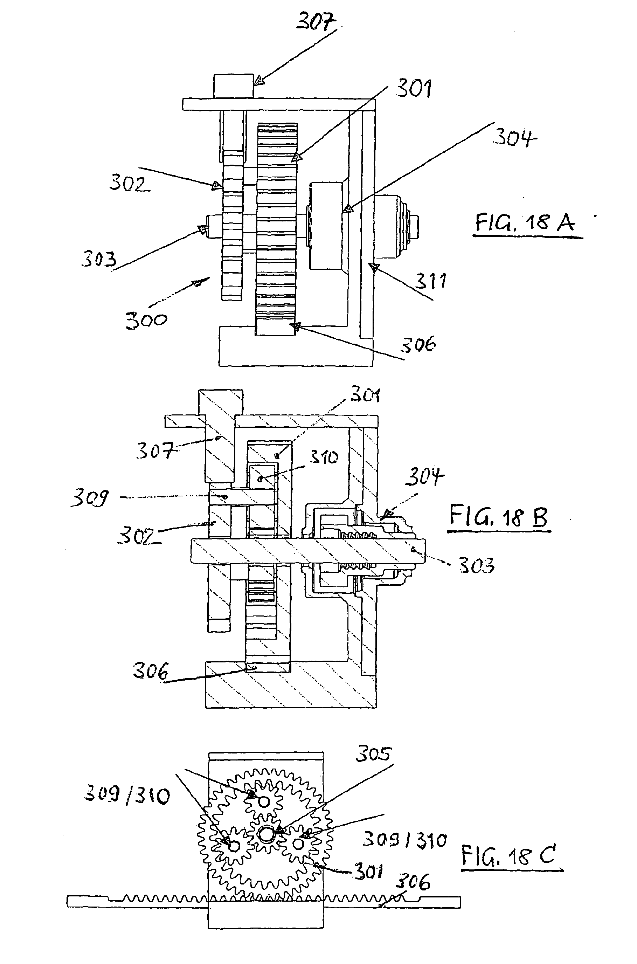

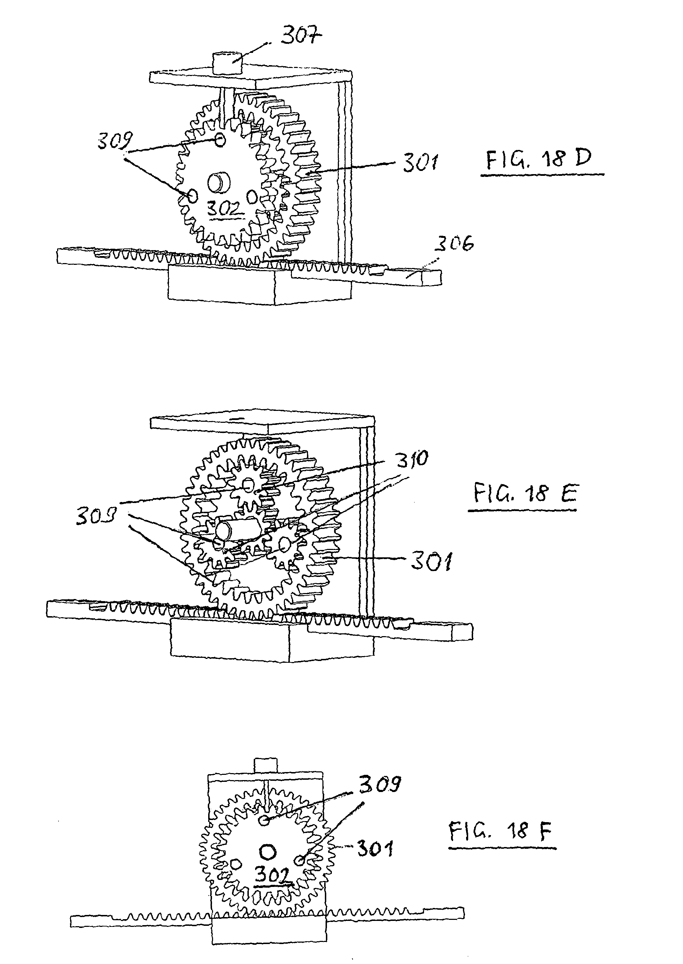

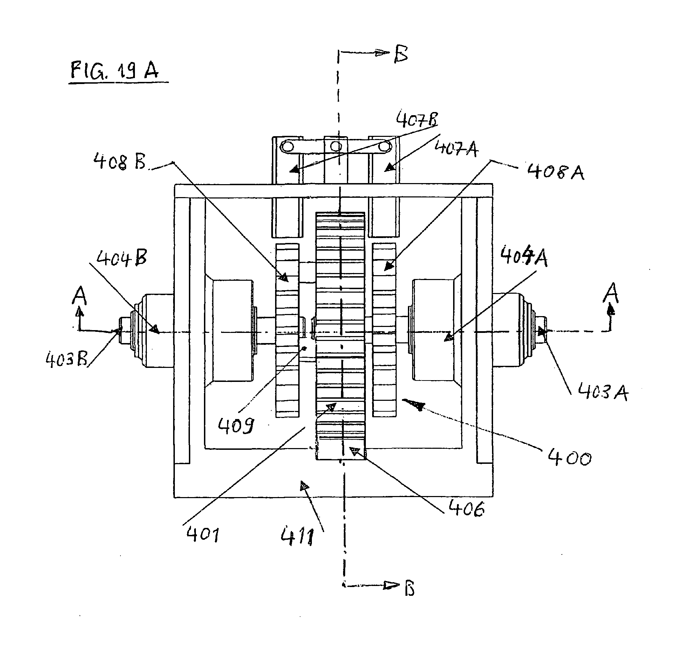

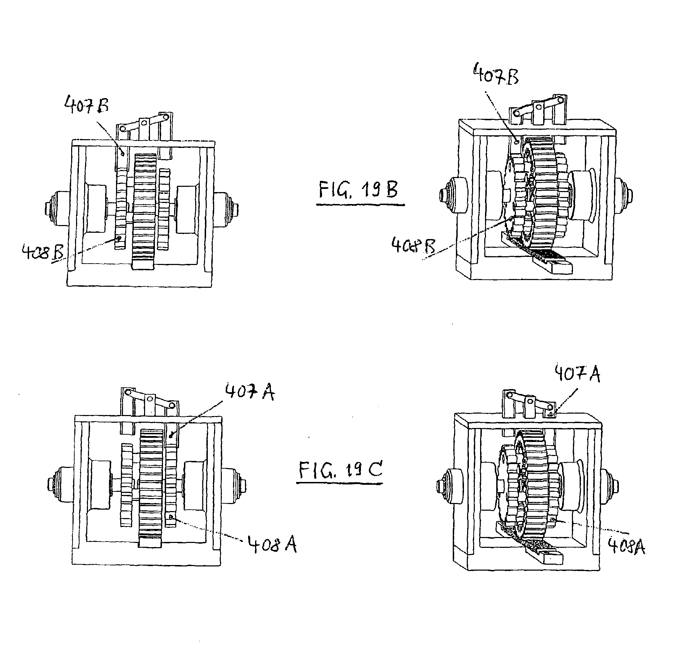

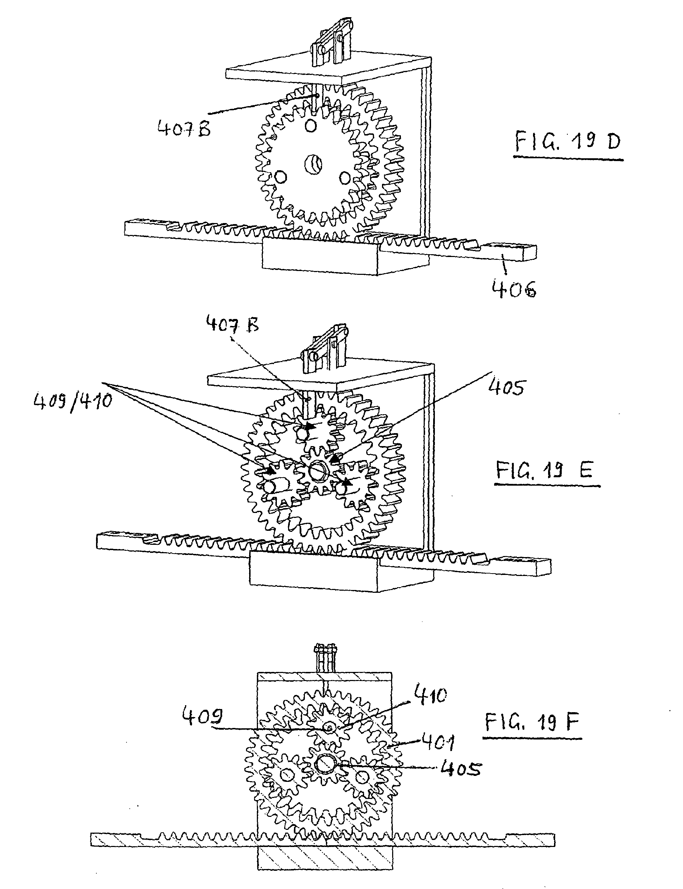

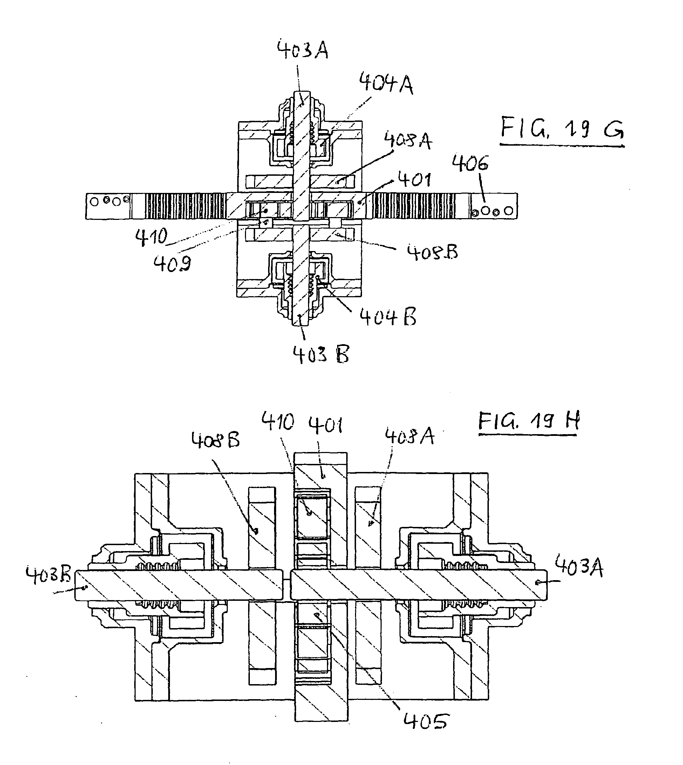

In den Figuren 18 und 19 sind zwei Ausführungsbeispiele eines Dämpfungsgliedes dargestellt, in denen die Kopplung der Zahnstange als Hubbestimmendes Bauteil mit einem/zwei Rotationsdämpfungsglied(er) über ein Planetengetriebe bewirkt wird, das mittels eines Sperrelements als Ein/Ausschalter des Rotationsdämpfungsgliedes konzipiert ist. Die Bauteile sind in einem Gehäuse 311,411 gelagert bzw. gehalten.

Das in den Figuren 18A-18F dargestellte erste Ausführungsbeispiel ist wie folgt aufgebaut:
Das Hohlrad 301 und der mit einer Umfangsverzahnung versehene Planetenradträger 302 eines Planetengetriebes 300 sind freilaufend auf der Welle 303 gelagert. Das Sonnenrad 305 ist fest mit der Welle 303 verpresst und wirkt somit als Antrieb für das mit der Welle 303 verbunde Rotationsdämpfungsglied 304.

Der Planetenradträger 302 ist über drei Achsen 309 mit den drei Planetenrädern 310 fest verbunden. Diese umlaufen das Sonnenrad 305 über eine Verzahnung. Über die Zahnstange 306 wird das Hohlrad 301 angetrieben. Ein Sperrschieber 307 kann die Drehung des Planetenradträgers 302 verhindern, indem er in dessen Verzahnung eingreift. Je nach Stellung des Sperrschiebers 307 werden die entsprechenden Zahnräder in folgenden Funktionen betrieben:
- Der Planetenradträger 302 ist nicht gesperrt (Figuren 18C,E):
   Der Sperrschieber 307 sperrt den Planetenradträger 302 nicht. Das Hohlrad 301 dreht sich, wodurch die Planetenräder 310 um das Sonnenrad 305 laufen und somit eine Drehung des Planetenradträgers 302 bewirken. Das Sonnenrad 305 wird somit nicht bewegt, weshalb das Rotationsdämpfungsglied 304 nicht angetrieben wird. Es erfolgt also ein Leerlauf, d.h., der mit der Zahnstange 306 gekoppelte Hub der Injektionsvorrichtung läuft ungedämpft mit maximaler Geschwindigkeit und somit in kürzester Zeit ab.
- Der Planetenradträger 302 ist gesperrt (Figuren 18A,B,D,F):
   Der Sperrschieber 307 greift in den Planetenradträger 302 ein. Das Hohlrad 301 dreht sich durch die lineare Bewegung der Zahnstange 306. Das Hohlrad 301 treibt die Planetenräder 310 an. Da der Planetenradträger 302 in seiner Stellung fixiert ist, können sich die einzelnen Planetenräder 310 nicht um das Sonnenrad 305 bewegen. Die Planetenräder 310 treiben somit das Sonnenrad 305 an. Da dieses über die Welle 303 mit dem Rotationsdämpfungsglied 304 verbunden ist, wird dieses angetrieben. Es erfolgt also eine Dämpfung, d.h. der mit der Zahnstange 306 gekoppelte Hub der Injektionsvorrichtung verläuft mit geringerer Geschwindigkeit/ in längerer Zeitspanne.

Bei Verwendung von zwei nacheinander gesetzten Rotationsdämpfungsgliedern mit unterschiedlichen Dämpfungswerten, die in dieser Weise betrieben werden, sind mehrere Kombinationen möglich:
1) Keiner der beiden Sperrschieber gedrückt → Leerlauf, keine Hubdämpfung,
2) nur ein Sperrschieber gedrückt → nur das zugehörige Rotationsdämfungsglied dämpft, Hubdämpfung entsprechend dem gewählten Rotationsdämpfungsglied,
3) beide Sperrschieber gedrückt → beide Rotationsdämpfungsglieder dämpfen, maximale Dämpfung des Hubs.

Das in den Figuren 19A-19H dargestellte zweite Beispiel ist eine Erweiterung des ersten Beispiels und wie folgt aufgebaut:
Es sind zwei Wellen 403A und 403B vorgesehen. Die erste Welle 403A dient als Antrieb des ersten Rotationsdämpfungsglieds 404A. Auf der ersten Welle 403A ist eine erste Sperrscheibe 408A fest angeordnet z.B. aufgepresst. Das Hohlrad 401 dreht sich freilaufend auf der ersten Welle 403A. Zudem ist das Sonnenrad 405 fest mit der ersten Welle 403A verbunden. Der Antrieb erfolgt über die Zahnstange 406, welche das Hohlrad 401 antreibt.

Die zweite Welle 403B dient als Antrieb für das zweite Rotationsdämfungsglied 404B. Auf der zweiten Welle 403B ist eine zweite Sperrscheibe 408B fest gelagert. Die zweite Sperrscheibe 408B dient als Planetenradträger, da über die drei Achsen 409 die drei Planetenräder 410 freilaufend verbunden sind. Die Planetenräder 410 werden über das Hohlrad 401 angetrieben und treiben je nach Stellung der Sperrschieber 407A,407B der Sperreinrichtung die entsprechenden Zahnräder an.

Somit ergeben sich folgende Funktionen:
- Die erste Sperrscheibe 408A ist gesperrt (Figur 19C):
   Die Sperreinrichtung sperrt über ihren ersten Sperrschieber 407A die Drehung der ersten Sperrscheibe 408A und damit die Drehung der ersten Welle 403A. Das Hohlrad 401 dreht sich durch die lineare Bewegung der Zahnstange 406 und treibt die Planetenräder 410 an.

Da die erste Welle 403A sich nicht drehen kann, ist das Sonnenrad 405 in seiner Stellung fixiert. Die Planetenräder 410 können sich somit um das Sonnenrad 405 bewegen und treiben so den Planetenradträger/zweite Sperrscheibe 408B an. Da diese über die zweite Welle 403B mit dem zweiten Rotationsdämpfungsglied 404B verbunden ist, wird das zweite Rotationsdämpfungsglied 404B angetrieben. Es erfolgt also eine Dämpfung durch das zweite Rotationsdämpfungsglied 404B. (Die zweite Sperrscheibe 408B dreht sich ohne Wirkung).
- Die zweite Sperrscheibe 408B ist gesperrt (Figuren 19B,D,E):
   Die Sperreinrichtung sperrt über ihren zweiten Sperrschieber 407B die Drehung der zweiten Sperrscheibe 408B und damit die Drehung der zweiten Welle 403B. Das Hohlrad 401 dreht sich durch die lineare Bewegung der Zahnstange 406 und treibt die Planetenräder 410 an. Da der Planetenradträger (= zweite Sperrscheibe 408B) in seiner Stellung fixiert ist, können sich die Planetenräder 410 nicht um das Sonnenrad 405 bewegen. Die Planetenräder 410 treiben somit das Sonnenrad 405 an. Da dieses über die erste Welle 403A mit dem ersten Rotationsdämpfungsglied 404A verbunden ist, wird dieses angetrieben. Es erfolgt also eine Dämpfung des Hubs durch das erste Rotationsdämpfungsglied 404A. (Die erste Sperrscheibe 408A dreht sich ohne Wirkung).

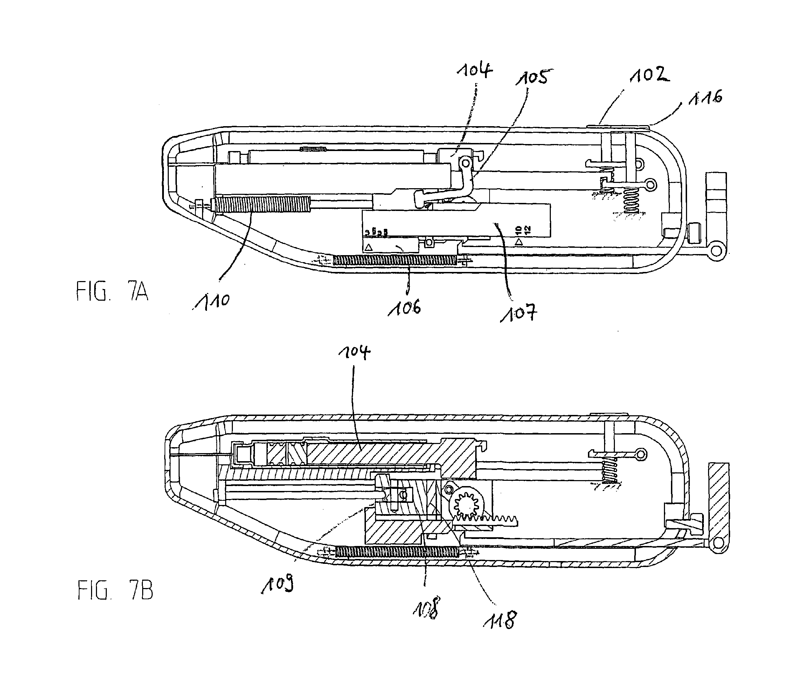

Die unter den Figuren 17 und 18 beschriebenen Baueinheiten mit den Bezeichnungen "Zahnstange 306,406 / Planetengetriebe 300,400 / Rotationsdämpfungsglied 304,404A,404B" können bei den in den Figuren 1 bis 16 gezeigten beiden Ausführungsbeispielen der Injektionsvorrichtung anstelle der dort gezeigten Baueinheiten mit den Bezeichnungen "Zahnstange 140,240,241 / Dämpfungsglied 150,250,251" eingesetzt werden.

Durch eine geeignete Verbindung der Sperrschieber mit Betätigungsmitteln außerhalb des Gehäuses kann vom Benutzer ein Dämpfungsglied aktiviert oder ausgewählt werden, um beispielsweise einen langsameren Injektionshub zu erreichen.

Der Patient erhält somit eine weitere Einstelloption zur individuellen Gestaltung der Ablaufprofile der Hube "seiner" Injektionsvorrichtung.

### Bezugszeichen

- Gehäuse: 101,201
- erste Taste: 102,202
- Rasthaken: 102A,202A
- Aufnahme: 103,203
- Stössel: 104,204
- Steuerhebel: 105,205
- erster Einstellschieber: 107,207
- zweiter Einstellschieber: 106,206
- Rampe: 106A206A
- Anschlag: 206B
- Schlitten: 108,208
- Rolle: 109,209
- Feder: 110,210
- Karpule: 111
- Spritze: 211
- Kanüle: 112,212
- Zugseil: 114,214
- Zugstange: 115,215
- zweite Taste: 116
- Rasthaken: 116A
- Rückzuggriff: 117,217
- Mitnehmer: 118,218
- Mitnehmerfeder: 119,219
- Rückzugfeder: 120,220
- Arretierung Aufnahme: 221
- Führung Arretierung: 222
- Rampe: 222A
- Feder: 223
- erste Einstellschraube: 130,230

- zweite Einstellschraube: 231
- erste Anschlagfläche: 130A,230A
- zweite Anschlagfläche: 231 A
- erste Zahnstange: 140,240
- zweite Zahnstange: 241
- erstes Dämpfungsglied: 150,250
- zweites Dämpfungsglied: 251
- Planetengetriebe: 300,400
- Hohlrad: 301,401
- Planetenradträger: 302,402
- Welle: 303,403A,403B
- Rotationsdämpfungsglied: 304,404A,404B
- Sonnenrad: 305,405
- Zahnstange: 306,406
- Sperrschieber: 307,407A,407B
- Sperrscheiben: 408A,408B
- Achsen: 309,409
- Planetenräder: 310,410
- Gehäuse: 311,411

## Patentansprüche

1. Injektionsvorrichtung zur Aufnahme und Betätigung einer Karpule (111) oder einer Spritze (211) mit einer Injektionsnadel, und mit Bauteilen, durch deren Relativbewegung der Ablauf der Injektion des Wirkstoff bewirkt wird, wozu in einem Gehäuse (101,201) eine Aufnahme (103, 203) gehalten ist, in die die Karpule/Spritze (111,211) einlegbar und fixierbar ist, die Aufnahme (103,203) mittels eines Schlittens (108,208) verschiebbar ist, in der Aufnahme (103, 203) ein Stössel (104, 204) verschiebbar gehalten ist, der den/die Kolben (111A,111B;211) der Karpule/Spritze beaufschlagt, und wobei zur Durchführung von Einstechhub, Injektionshub und Rückholhub ein Zugseil (114,214) vorgesehen ist, das über eine am Schlitten (108, 208) gelagerte Rolle (109, 209) umgelenkt wird, und dessen eines Ende mit der Aufnahme (103, 203) und dessen anderes Ende mit einer Zugfeder (110, 210) verbunden ist, die am Gehäuse (101, 201) gehalten ist, wobei automatisch und/oder manuell betätigbare Einrichtungen zwischen Gehäuse (101,201), Aufnahme (103, 203), Stössel (104, 204) und Schlitten (108, 208) deren wechselseitige Kopplung mit dem Zugseil (114, 214) und damit die Abfolge von Einstechhub, Injektionshub und Rückholhub steuern, **dadurch gekennzeichnet, dass** die Einrichtungen zumindest ein in seiner Position verstellbares Mittel zur benutzerseitigen Einstellung des Ablaufprofils zumindest eines der Hube enthalten.

2. Injektionsvorrichtung nach Anspruch 1, bei der das Zugseil (114, 214) über den Schlitten (108, 208) und die Rolle (109, 209) bei dem auf den Injektionshub folgenden Rückholhub die Aufnahme (103, 203) mit der Karpule/Spritze und der Injektionsnadel aus der Einstichstelle zieht, **dadurch gekennzeichnet, dass** mit mindestens einem ersten Mittel die Dauer eines bewegungsfreien Zustands der Karpule/Spritze zwischen Injektionshub und Rückholhub (Leerhub HX) am Gehäuse (101, 201) einstellbar ist, während dem die Injektionsnadel in der Einstichstelle verbleibt.

3. Injektionsvorrichtung nach Anspruch 1, wobei Einrichtungen zur Koppelung des Stössels (104, 204) mit der Aufnahme (103, 203) vorgesehen sind, die den Stössel (104, 204) zur Durchführung des Einstechhubs mit der Aufnahme (103, 203) koppeln und zur Durchführung des Injektionshubs entkoppeln, **dadurch gekennzeichnet, dass** mit mindestens einem zweiten Mittel der Zeitraum zwischen Beginn und Ende der Bewegung des Stössels (104, 204) in der Aufnahme (103, 203) und damit die Dauer des Injektionshubs (H2) einstellbar ist.

4. Injektionsvorrichtung nach Anspruch 2 oder 3, **dadurch gekennzeichnet, dass** das erste und/oder zweite Mittel eine verschiebbar gelagerte Zahnstange (140,240,241) beinhaltet.

5. Injektionsvorrichtung nach Anspruch 2 oder 3, **dadurch gekennzeichnet, dass** das erste und/oder zweite Mittel mindestens ein Dämpfungsglied (150,250,251) beinhaltet, das von einer zugeordneten Zahnstange (140,240,241) betätigbar ist.

6. Injektionsvorrichtung nach Anspruch 4 und 5, **dadurch gekennzeichnet, dass** die Wirkdauer des Dämpfungsgliedes (150,250,251) über einen Abschnitt des zugeordneten Hubs (HX,H2) mittels Stellelementen (130,230,231) am Gehäuse einstellbar ist, die die Verschiebung der zugeordneten Zahnstange (140,240,241) zwischen zwei Endpositionen bewirken.

7. Injektionsvorrichtung nach Anspruch 6, **dadurch gekennzeichnet, dass** das Dämpfungsglied eine am Gehäuse einstellbarer Dämpfungscharakteristik aufweist.

8. Injektionsvorrichtung nach Anspruch 5, **dadurch gekennzeichnet, dass** das Dämpfungsglied ein Rotationsdämpfungsglied (304,404A,404B) mit einem Zahnrad auf seiner Welle ist, das von einer Zahnstange (140,240,241) beaufschlagt wird.

9. Injektionsvorrichtung nach Anspruch 8, **dadurch gekennzeichnet, dass** zwischen einem Rotationsdämpfungsglied und seiner Zahnstange ein Getriebe angeordnet ist.

10. Injektionsvorrichtung nach Anspruch 9, **dadurch gekennzeichnet, dass** das Getriebe ein Planetengetriebe (300) ist, dessen Sonnenrad (305) fest mit der Welle (303) des Rotationsdämpfungsglieds (304) verbunden ist, dessen auf der Welle (303) frei drehbares Hohlrad (301), in dem Planetenräder (310) umlaufen, mit der Zahnstange kämmt, und dessen auf der Welle (303) frei drehbarer Planetenradträger (302) in Eingriff mit einem vom Benutzer betätigbaren Sperrschieber (307) bringbar ist, bei dem dieser die Drehung des Planetenradträgers (302) verhindert.

11. Injektionsvorrichtung nach Anspruch 10, **dadurch gekennzeichnet, dass** zwei Rotationsdämpfungsglieder (404A,404B) auf miteinander konzentrisch gelagerten Wellen (403A,403B) gehalten sind, wobei die erste Welle (403A) fest mit einer ersten Sperrscheibe (408A) und dem Sonnenrad (405) verbunden ist, und sich das Hohlrad (401) auf dieser ersten Welle (403A) frei dreht, wogegen die zweite Welle (403B) fest mit dem als zweite Sperrscheibe (408B) ausgebildeten Planetenradträger verbunden ist, und dass ein vom Benutzer einstellbares Sperrelement alternativ die Drehung einer der beiden Sperrscheiben (408A,408B) verhindern kann.

12. Injektionsvorrichtung nach Anspruch 1, **dadurch gekennzeichnet, dass** bei Verwendung einer Karpule (111) dem Einstechhub ein Mischhub vorgeschaltet ist

## Claims

1. An injection device for accommodation and activation of a carpule (111) or a hypodermic syringe (211) with an injection needle, and incorporating components whose relative movement effects the sequence of injection of the active substance, for which purpose a receptacle (103, 203), into which the carpule /syringe (111, 211) is insertable and in which it is fixable, is supported inside a housing (101, 201), and said receptacle (103, 203) is displaceable by means of a carriage (108, 208), and mounted in the receptacle (103, 203) in a manner so as to be displaceable is a ram (104, 204), which actuates the piston(s) (111A, 111 B; 211) of the carpule/syringe, and wherein a traction cable (114, 214) is provided for carrying out the puncture stroke, injection stroke and a retraction stroke, which is deflected by means of a roller (109, 209) that is mounted on the carriage (108, 208), one end of which is connected to the receptacle (103, 203) and the other end of which is connected to a tension spring (110, 210), the latter being supported on the housing (101, 201), wherein automatically and/or manually activated devices between the housing (101, 201), receptacle (103, 203), ram (104, 204) and carriage (108, 208) control their alternating coupling to the traction cable (114, 214) and thereby the progression of puncture stroke, injection stroke and retraction stroke, **characterized in that** the devices include at least one means that can be adjusted in its position for adjustment of the progression profile of at least one of the strokes by the user.

2. An injection device according to claim 1, wherein the traction cable (114, 214) pulls the receptacle (103, 203) with the carpule/syringe and pulls the injection needle out of the puncture site via the carriage (108, 208) and roller (109, 209) during the retraction stroke following the injection stroke, **characterized in that** the duration of a movement-free state of the carpule/syringe between the injection stroke and retraction stroke (idle stroke HX), during which the injection needle remains in the puncture site, is adjustable at the housing (101, 201) by means of at least one first means.

3. An injection device according to claim 1, wherein devices are provided for coupling the ram (104, 204) to the receptacle (103, 203), which couple the ram (104, 204) to the receptacle (103, 203) for carrying out the puncture stroke and uncouple it for carrying out the injection stroke, **characterized in that** the duration between the beginning and end of the movement of the ram (104, 204) in the receptacle (103, 203) and thereby the duration of the injection stroke (H2) is adjustable by means of at least one second means.

4. An injection device according to claim 2 or 3, **characterized in that** the first and/or second means includes a displaceably supported toothed rack (140, 240, 241).

5. An injection device according to claim 2 or 3, **characterized in that** the first and/or second means includes at least one damping element (150, 250, 251) that is actuatable from an associated toothed rack (140, 240, 241).

6. An injection device according to claim 4 and 5, **characterized in that** the duration of the effect of the damping element (150, 250, 251) is adjustable at the housing via a portion of the associated stroke (HX, H2) by means of positioners (130, 230, 231) that effect the displacement of the associated toothed rack (140, 240, 241) between two end positions.

7. An injection device according to claim 6, **characterized in that** the damping element has a damping characteristic that is adjustable at the housing.

8. An injection device according to claim 5, **characterized in that** the damping element is a rotation damping element (304, 404A, 404B) having a gear wheel on its shaft that is acted upon by a toothed rack (140, 240, 241).

9. An injection device according to claim 8, **characterized in that** there is disposed between a rotation damping element and its toothed rack a gear mechanism.

10. An injection device according to claim 9, **characterized in that** the gear mechanism is a planetary gear (300), whose sun gear (305) is rigidly connected to the shaft (303) of the rotation damping element (304), whose annulus gear (301), which is freely rotatable on the shaft (303) and in which planetary gears (310) revolve, intermeshes with the toothed rack, and whose planetary gear carrier (302), which is freely rotatable on the shaft (303), can be put into engagement with a user-activated blocking slide (307), wherein the same prevents the rotation of the planetary gear carrier (302).

11. An injection device according to claim 10, **characterized in that** two rotation damping elements (404A, 404B) are held on shafts (403A, 403B), that are disposed concentrically to each other, wherein the first shaft (403A) is rigidly connected to a first blocking disc (408A) and to the sun gear (405), and the annulus gear (401) freely rotates about this first shaft (403A), whereas the second shaft (403B) is rigidly connected to the planetary gear carrier that is designed so as to form the second blocking disc (408B), and that a user-adjustable blocking element can alternatively block the rotation of one of the two blocking discs (408A, 408B).

12. An injection device according to claim 1, **characterized in that** when a carpule (111) is used, the puncture stroke is preceded by a mixing stroke.

## Revendications

1. Dispositif d'injection pour recevoir et actionner une carpule (111) ou une seringue (211) comprenant une aiguille d'injection, et comprenant des éléments par le mouvement relatif desquels s'effectue le déroulement de l'injection de substance active, à cette fin un logement (103, 203) dans lequel la carpule/seringue (111, 211) est insérable et fixable, est maintenu dans un boîtier (101, 201) et déplaçable au moyen d'un chariot (108, 208), un poussoir (104, 204) agissant sur le(s) piston(s) (111A, 111B ; 211) de la carpule/seringue est maintenu déplaçable dans le logement (103, 203), et il est prévu, pour la mise en oeuvre d'une course de piqûre, course d'injection, et course de retour, un cordon tracteur (114, 214) qui est dévié par l'intermédiaire d'un galet (109, 209) monté sur le chariot (108, 208), et dont une extrémité est reliée au logement (103, 203) et l'autre extrémité est reliée à un ressort de traction (110, 210) qui est tenu au boîtier (101, 201), et des dispositifs actionnables manuellement et/ou automatiquement entre le boîtier (101, 201), le logement (103, 203), le poussoir (104, 204) et le chariot (108, 208) pilotent leur couplage réciproque avec le cordon tracteur (114, 214), et ainsi le déroulement de la course de piqûre, la course d'injection et la course de retour, **caractérisé en ce que** les dispositifs comportent au moins un moyen réglable dans sa position pour le réglage côté utilisateur du profil de déroulement d'au moins une des courses.

2. Dispositif d'injection selon la revendication 1, dans lequel lors de la course de retour faisant suite à la course d'injection, le cordon tracteur (114, 214) tire le logement (103, 203) avec la carpule/seringue et l'aguille d'injection, hors de l'emplacement de la piqûre, par l'intermédiaire du chariot (108, 208) et du galet (109, 209), **caractérisé en ce que** la durée d'un état d'absence de mouvement de la carpule/seringue entre la course d'injection et la course de retour (course à vide HX) est réglable sur le boîtier (101, 201) avec au moins un premier moyen, pendant lequel l'aiguille d'injection reste dans l'emplacement de la piqûre.

3. Dispositif d'injection selon la revendication 1, dans lequel, pour coupler le poussoir (104, 204) avec le logement (103, 203), il est prévu des dispositifs qui couplent le poussoir (104, 204) avec le logement (103, 203) pour la mise en oeuvre de la course de piqûre, et les découplent pour la mise en oeuvre de la course d'injection, **caractérisé en ce que** l'intervalle de temps entre le début et la fin du mouvement du poussoir (104, 204) dans le logement (103, 203), et ainsi la durée de la course d'injection (H2), est réglable avec au moins un deuxième moyen.

4. Dispositif d'injection selon la revendication 2 ou 3, **caractérisé en ce que** le premier et/ou le deuxième moyen comporte une crémaillère (140, 240, 241) montée déplaçable.

5. Dispositif d'injection selon la revendication 2 ou 3, **caractérisé en ce que** le premier et/ou le deuxième moyen comporte au moins un élément d'amortissement (150, 250, 251) qui est actionnable par une crémaillère (140, 240, 241) associée.

6. Dispositif d'injection selon la revendication 4 ou 5, **caractérisé en ce que** la durée d'action de l'élément d'amortissement (150, 250, 251) est réglable sur un tronçon de la course (HX, H2) associée, au moyen d'éléments de réglage (130, 230, 231) sur le boîtier qui provoquent le déplacement de la crémaillère (140, 240, 241) associée entre deux positions extrêmes.

7. Dispositif d'injection selon la revendication 6, **caractérisé en ce que** l'élément d'amortissement présente une caractéristique d'amortissement réglable sur le boîtier.

8. Dispositif d'injection selon la revendication 5, **caractérisé en ce que** l'élément d'amortissement est un élément d'amortissement de rotation (304, 404A, 404B) comprenant une roue dentée sur son arbre, sur laquelle agit une crémaillère (140, 240, 241).

9. Dispositif d'injection selon la revendication 8, **caractérisé en ce qu'**un engrenage est agencé entre un élément d'amortissement de rotation et sa crémaillère.

10. Dispositif d'injection selon la revendication 9, **caractérisé en ce que** l'engrenage est un engrenage planétaire (300), dont la roue planétaire (305) est solidaire de l'arbre (303) de l'élément d'amortissement de rotation (304), dont la couronne (301) librement rotative sur l'arbre (303) dans laquelle orbitent des satellites (310), engrène avec la crémaillère, et dont un porte-satellites (302) librement rotatif sur l'arbre (303) peut être amené en prise avec un poussoir de blocage (307) actionnable par l'utilisateur, celui-ci empêchant alors la rotation du porte-satellites (302).

11. Dispositif d'injection selon la revendication 10, **caractérisé en ce que** deux éléments d'amortissement de rotation (404A, 404B) sont tenus sur des arbres (403A, 403B) concentriques l'un à l'autre, le premier arbre (403A) étant solidaire du premier disque de blocage (408A) et de la roue planétaire (405), et la couronne (401) tournant librement sur ce premier arbre (403A), tandis que le deuxième arbre (403B) est solidaire du porte-satellites formant deuxième disque de blocage (408B), et **en ce qu'**un élément d'arrêt réglable par l'utilisateur peut empêcher au choix la rotation de l'un des deux disques de blocage (408A, 408B).

12. Dispositif d'injection selon la revendication 1, **caractérisé en ce que** lors de l'utilisation d'une carpule (111) une course de mélange est placée en amont de la course de piqûre.
